**Europäisches Patentamt**

**European Patent Office**

**Office européen des brevets**

(19)

(11) Veröffentlichungsnummer: **0 195 080**
**B1**

(12) # EUROPÄISCHE PATENTSCHRIFT

(45) Veröffentlichungstag der Patentschrift:
23.11.88

(51) Int. Cl.⁴: **A 61 F 5/46**

(21) Anmeldenummer: **85905048.6**

(22) Anmeldetag: **17.09.85**

(86) Internationale Anmeldenummer:
**PCT/DE 85/00327**

(87) Internationale Veröffentlichungsnummer:
**WO 86/01708 (27.03.86 Gazette 86/07)**

(54) VORRICHTUNG ZUM EINFÜHREN EINES DIAPHRAGMAS.

(30) Priorität: **21.09.84 DE 3434721**

(43) Veröffentlichungstag der Anmeldung:
**24.09.86 Patentblatt 86/39**

(45) Bekanntmachung des Hinweises auf die Patenterteilung:
**23.11.88 Patentblatt 88/47**

(84) Benannte Vertragsstaaten:
**AT BE CH DE FR GB IT LI LU NL SE**

(56) Entgegenhaltungen:
**US-A-2 008 380**
**US-A-4 398 532**

(73) Patentinhaber: **LEUCHTENBERGER, Hannegret, c/o Eckart Kück Poelzigstrasse 9, D-2800 Bremen 1 (DE)**

(72) Erfinder: **LEUCHTENBERGER, Hannegret, c/o Eckart Kück Poelzigstrasse 9, D-2800 Bremen 1 (DE)**

(74) Vertreter: **Pfenning, Meinig & Partner, Kurfürstendamm 170, D-1000 Berlin 15 (DE)**

EP 0 195 080 B1

## Beschreibung

Die Erfindung betrifft eine Vorrichtung zum Einführen eines Diaphragmas in eine Scheide sowie zum Plazieren des Diaphragmas vor der Gebärmutter, im wesentlichen bestehend aus einem hohlen Einführstab, an dessen in Längsrichtung geschlitzt ausgebildetem offenen Einführende das Diaphragma lösbar zu befestigen ist, und in dem ein relativ zum Einführstab verschiebbarer Kolben angeordnet ist.

Bei einem derartigen Diaphragma handelt es sich bekanntlich um ein Schwangerschafts-Verhütungsmittel, welches in die Scheide eingeführt und so plaziert wird, daß es sie von der Gebärmutter abschließt. Hierdurch ist es den männlichen Samenfäden nicht möglich, in die Eileiter zu gelangen, so daß eine Befruchtung nicht erfolgen kann. Zusätzlich zu diesem gleichsam mechanischen Schutz, wird darüber hinaus eine spermizide Creme, ein spermizider Schaum oder ein spermizides Gel verwandt, welche die männlichen Samenfäden innerhalb kürzester Zeit befruchtungsunfähig machen. Zum Einführen kann das sowohl im Bevorratungszustand als auch im plazierten Zustand in der Draufsicht kreisförmige, geometrisch kugelkalottenförmig ausgebildete Diaphragma, welches aus einer am Randabschnitt wulstartig verstärkten elastischen Membran besteht, durch seitliches Zusammendrücken des Wulstes an zwei einander gegenüberliegenden Punkten verformt werden, wobei die hierdurch von selbst entstehenden beiden Taschen (und ggf. der wulstartige Rand) mit spermizider Creme oder dgl. gefüllt bzw. bestrichen werden.

Obwohl dieses Verhütungsmittel seit vielen Jahren bekannt ist, hat es sich bisher noch nicht in dem seiner Bedeutung zukommenden Rahmen in praxi durchgesetzt. Dieses liegt insbesondere daran, daß das Einführen eines Diaphragmas ohne irgendwelche Hilfsmittel häufig Schwierigkeiten bereitet. Es kommt hinzu, daß das Bestreichen bzw. teilweise Füllen mit spermizider Creme oder dgl. dazu führt, daß sowohl Finger als auch die Scheide zwangsläufig mit spermizidem Mittel verschmiert werden, was als unangenehm bzw. störend empfunden wird.

Um das Einführen eines Diaphragmas zu erleichtern, ist ein Einführstab entwickelt und bekannt geworden, der an seinem einen Ende eine leichte Einkerbung aufweist, an welcher ein Diaphragma vor dem Einführen aufzuspannen ist. Zwecks derartiger Aufspannung sind im mittleren Bereich des bekannten Einführstabes weitere Einkerbungen vorhanden. An einer dieser im mittleren Bereich des Einführstabes vorhandenen Einkerbungen (die für verschiedene Diaphragmagrößen vorgesehen sind) kann sodann der gegenüberliegende Abschnitt des wulstartigen Randes so aufgespannt werden, wie dieses weiter oben im Zusammenhang mit einer Einführung von Hand beschrieben ist. Dabei wird bei dem bekannten Einführstab das Diaphragma so in die Hand genommen, daß seine Wölbung nach außen zeigt, und wie oben beschrieben seitlich zusammengedrückt. Nach dem Aufspannen am Einführende und im mittleren Abschnitt des Einführstabes bilden sich mithin dann die beiden oben bereits im Zusammenhang mit einer Einführung von Hand beschriebenen Taschen, die mit spermizider Creme oder dgl. ausgefüllt werden, wobei darüber hinaus empfohlen wird, auch den wulstartigen Rand mit der Creme oder dgl. zu bestreichen, um die Gleitfähigkeit beim Einführen zu verbessern.

Zum Einführen und Plazieren des Diaphragmas wird der Einführstab mit dem aufgespannten und entsprechend präparierten Diaphragma dicht am unteren Scheidenboden entlang bis hinter den Muttermund eingeführt und sodann zur Seite gedreht, um das Diaphragma vom Einführstab zu lösen und den Einführstab danach aus der Scheide herausziehen zu können. Anschließend ist der vordere Rand des Diaphragmas mit einem Finger so nach oben zu drücken, daß es sich hinter das Schambein setzt, wonach es so sitzen soll, daß der Muttermund vollständig bedeckt ist.

Es ist ohne weiteres erkennbar, daß mit dem bekannten Einführstab als Einführhilfe die Vorbehalte gegenüber diesem an sich äußerst zuverlässigen und verträglichen Verhütungsmittel nicht zu beheben waren, da die gesamte Handhabung nach wie vor recht kompliziert ist und schon deswegen von einem beträchtlichen Teil potentieller Anwenderinnen abgelehnt wird.

Es kommt hinzu, daß sich auch bei Verwendung dieses Einführstabes nicht verhindern läßt, daß ein beträchtlicher Teil der spermiziden Creme oder dgl. an Stellen gelangt, wo sie nicht erforderlich ist und als unangenehm und störend empfunden wird.

Um das Einführen zu erleichtern, ist aus der US-PS-2 008 380 eine gattungsgemäße Vorrichtung bekanntgeworden, bei welcher das Einführende des Einführstabes als gegenüber dem übrigen Abschnitt des Einführstabes erheblich verdickter Kopf ausgebildet ist, der etwa so lang ist wie der Radius eines Diaphragmas, so daß dieses zum Befestigen auf seiner einen Hälfte zusammengedrückt und in den Einführknopf eingeführt werden kann, während es mit seiner anderen Hälfte in einem bezüglich seiner Normalform nur teilweise verformten Zustand aus der Vorrichtung vorsteht. Da ein Diaphragma flexibel ist, kann es bei dieser bekannten Vorrichtung beim Einführen leicht seitlich abknicken bzw. unter ungünstigen Umständen überhaupt nicht in die Vagina eingeführt werden, wobei selbst unter relativ günstigen Einführbedingungen eine ordnungsgemäße genaue Plazierung vor der Gebärmutter äußerst schwierig ist.

Es kommt hinzu, daß das mit einem spermiziden Mittel versehene Diaphragma bei Verwendung dieser Vorrichtung beim Einführen ebenfalls zwangsläufig mit den Scheidenwänden in Berührung kommt, so daß es auch hier zwangsläufig zu einer Übertragung eines Teils des spermiziden Mittels auf die Scheidenwände kommt.

Weiterhin ist es bei dieser bekannten Vorrichtung nachteilig, daß sich an ihrer Unterseite ein an sich funktionsloser Schlitz befindet, der beim Einführen zu Reizungen oder gar Verletzungen des Scheidengewebes führen kann.

Aus der US-PS-2 141 040 ist eine aus einem zylindrischen Einführstab und einem im Querschnitt zylindrischen Kolben bestehende Vorrichtung zum Einführen eines Diaphragmas bekanntgeworden, die ebenfalls bezüglich des Einführens unzweckmäßig ist, da das Diaphragma vor Aufnahme in die Vorrichtung mit dem spermiziden Mittel versehen werden muß, so daß zwangsläufig auch an die Finger der Benutzerin spermizides Mittel gelangt. Weiterhin ist diese Vorrichtung nicht nur anatomisch ungünstig, sondern auch deshalb, weil die Benutzerin von außen nicht erkennen kann, in welcher relativen Drehstellung zur Längsachse sie den Einführstab einführen soll.

Aus der US-PS-4 398 532 ist eine Vorrichtung zum Einführen eines Diaphragmas mit einem hohlen Einführstab und einem Kolben bekanntgeworden, bei welcher der Einführstab relativ flach ausgebildet ist und sowohl das Diaphragma als auch spermizides Mittel enthält, wobei das Einführende zunächst durch eine Verschlußkappe verschlossen ist. Diese bekannte Vorrichtung ist nur zur Einmalverwendung bestimmt und geeignet, was im Hinblick auf die erheblichen Kosten eines Diaphragmas für eine verbreitete Anwendung kostenmäßig nicht vertretbar ist.

Aus der DE-OS-1 766 697 ist schließlich noch eine Vorrichtung zum Einführen eines Diaphragmas bekannt, bei welcher im Einführendabschnitt eines hohlen Einführstabes ein mehrfach zusammengefaltetes bzw. -gerolltes Diaphragma angeordnet ist, welches sich nach dem Ausstoßen so entfalten soll, daß es sich vor der Gebärmutter ordnungsgemäß plaziert. Auch bei dieser vorbekannten Vorrichtung muß das Diaphragma zunächst mit spermizidem Mittel versehen werden, wobei es verstärkt zu einer Übertragung von spermizidem Mittel auf die Finger der Benutzerin kommt, da das Diaphragma nicht nur seitlich zusammengedrückt, sondern noch darüber hinaus verformt werden soll, was im übrigen auch für eine ordnungsgemäße Plazierung vor der Gebärmutter nachteilig ist.

Der vorliegenden Erfindung liegt die Aufgabe zugrunde, die bekannten Vorrichtungen zum Einführen eines Diaphragmas unter Vermeidung ihrer Nachteile so zu verbessern, daß ein Einführen des von relativ ungeschickten bzw. nicht sachkundigen Anwenderinnen selbst bei Erstanwendung mühelos und ohne Schwierigkeiten so möglich ist, daß das Diaphragma ordnungsgemäß vor der Gebärmutter plaziert wird, wobei zugleich sichergestellt werden soll, daß diejenigen Vaginalbereiche, an denen kein spermizides Mittel erforderlich ist, ebenso wie die Finger einer Anwenderin, nicht mit spermizidem Mittel in Berührung kommen. Dabei soll die Vorrichtung beliebig häufig wiederverwendbar und demgemäß einfach zu reinigen sein.

Die Lösung dieser Aufgabe ist erfindungsgemäß dadurch gekennzeichnet, daß der lichte Querschnitt des Einführstabes durch einen abgerundeten Boden und einen hieran anschließenden, verbreiterten abgerundeten Randbereich zum geführten Halten des Diaphragma-Randwulstes hutförmig ausgebildet ist, und der Einführstab mit einem einzigen, im verbreiterten Randbereich angeordneten Schlitz versehen ist.

Für eine Versorgung des Diaphragmas mit einem spermiziden Mittel kann dieses beispielsweise aus einer Tube in die nischeartig verbreiterten Randbereiche eingegeben werden, die zur Aufnahme des wulstartig verdickten Diaphragmas dienen, bevor das Diaphragma in einen seitlich zusammengedrückten Zustand vom offenen Ende des Einführendes her in den hohlen Einführstab eingeschoben wird. Sodann können beispielsweise streifenförmige Bereiche des durch den Schlitz zugänglichen Bereiches des Diaphragmas mit spermizidem Mittel versehen werden, wobei das Diaphragma in diesem seitlich zusammengedrückten Zustand aufgrund der hutförmigen Querschnittsgestaltung nicht nach oben aus dem Schlitz austritt, sondern sich mit seinen Falten nach unten in den Bodenbereich erstreckt, so daß es beim Einführen nicht dazu kommt, daß das spermizide Mittel an die Vaginalwände gelangen kann. Erst nach vollständigem Einführen des Einführstabes in die Scheide wird dieser relativ zu dem kolbenartigen Einsatz zurückgezogen, so daß dabei das Diaphragma an der Gebärmutter ordnungsgemäß frei wird und zu plazieren ist.

Obwohl es hierfür grundsätzlich ausreicht, wenn der Schlitz sich lediglich über eine Länge erstreckt, die etwa der Länge des Diaphragmas in seinem seitlich zusammengedrückten Zustand entspricht, besteht eine bevorzugte Ausgestaltung darin, daß sich der Schlitz über die gesamte Länge des Einführstabes erstreckt. Eine solche Ausgestaltung hat nämlich den weiteren Vorteil, daß der Kolben relativ kurz ausgebildet sein kann, wobei bei dieser Ausgestaltung zugleich zwangsläufig sichergestellt wird, daß das Diaphragma nicht mittels des Kolbens aus dem Einführstab ausgestoßen wird, sondern daß nach einem vollständigen Einführen des Einführstabes in die Scheide der Kolben mit einem Finger festgehalten und der Einführstab relativ zu dem Kolben zurückgezogen wird, um das Diaphragma aus dem Hohlraum des Einführstabes zu befreien und vor der Gebärmutter zu plazieren.

Ein weiterer Vorteil dieser Ausgestaltung besteht darin, daß sich der Einführstab nach Gebrauch beispielsweise mit einer seinem Querschnittsprofil angepaßten Bürste oder dgl. in wirkungsvoller und doch einfacher Weise sehr gut reinigen läßt.

Es hat sich als besonders zweckmäßig erwiesen, wenn der Schlitz zum Einführende hin erweitert ausgebildet ist. Eine derartige Erweiterung, die nicht notwendigerweise mit einer Querschnittsvergrößerung des Einführstabes einhergehen muß, erleichtert sowohl das Einführen des seitlich zusammengedrückten Diaphragmas in den Einführstab wie auch das Ausschieben des Diaphragmas aus dem Einführstab.

Für eine möglichst optimale und gleichsam selbsttätige Plazierung an der richtigen Stelle unter gleichzeitiger Berücksichtigung der anatomischen Verhältnisse hat es sich weiterhin als besonders zweckmäßig erwiesen, wenn das Einführende des Einführstabes leicht gekrümmt ausgebildet ist, wie weiter unten anhand von Ausführungsbeispielen noch weiter erläutert ist.

Bevorzugte Ausgestaltungen der vorliegenden Erfindung sind in den Unteransprüchen beschrieben.

Die Erfindung ist nachstehend an Ausführungsbeispielen unter Bezugnahme auf eine Zeichnung weiter erläutert. Es zeigt:

Fig. 1      eine seitliche Draufsicht auf eine erfindungsgemäße Vorrichtung in Explosionsdarstellung, d. h. mit aus dem hohlen Einführstab herausgezogenem Kolben in Richtung des Pfeiles 1 in Fig. 2 gesehen;

Fig. 2      eine seitliche Draufsicht auf die Vorrichtung gemäß Fig. 1 in Richtung des Pfeiles 11 in Fig. 1 gesehen;

Fig. 3      eine stirnseitige Draufsicht vom Einführende her auf die Vorrichtung gemäß den Fig. 1 und 2 in Richtung des Pfeiles III in Fig. 1 gesehen;

Fig. 4      eine stirnseitige Draufsicht auf die Vorrichtung gemäß den Fig. 1 bis 3 in Richtung des Pfeiles IV in Fig. 1 gesehen;

Fig. 5      einen Schnitt durch den kolbenartigen Einsatzkörper in Richtung der Schnittlinie V-V gesehen (unter Fortlassung der am oberen Ende des Kolbens vorhandenen Handhabe);

Fig. 6      einen Schnitt durch den Einführstab (ohne Kolben und unter Fortlassung der Handhabe des Einführstabes) in Richtung der Schnittlinie VI-VI gesehen;

Fig. 7      eine Fig. 6 entsprechende Darstellung in Richtung der Schnittlinie VII-VII gesehen,

Fig. 8      eine Variante in einer Darstellung gemäß Fig. 1 in Richtung des Pfeiles VIII in Fig. 9 gesehen;

Fig. 9      eine seitliche Draufsicht auf die Vorrichtung gemäß Fig. 8 in Richtung des Pfeiles IX in Fig. 8 gesehen;

Fig. 10      eine stirnseitige Draufsicht auf die Vorrichtung gemäß den Fig. 8 und 9 vom Einführende her in Richtung des Pfeiles X gesehen;

Fig. 11      eine stirnseitige Draufsicht auf die Vorrichtung gemäß den Fig. 8 bis 10 in Richtung des Pfeiles XI in Fig. 8 gesehen; und

Fig. 12      einen Schnitt durch den Einsatzkörper in Richtung der Schnittlinie XII-XII gesehen.

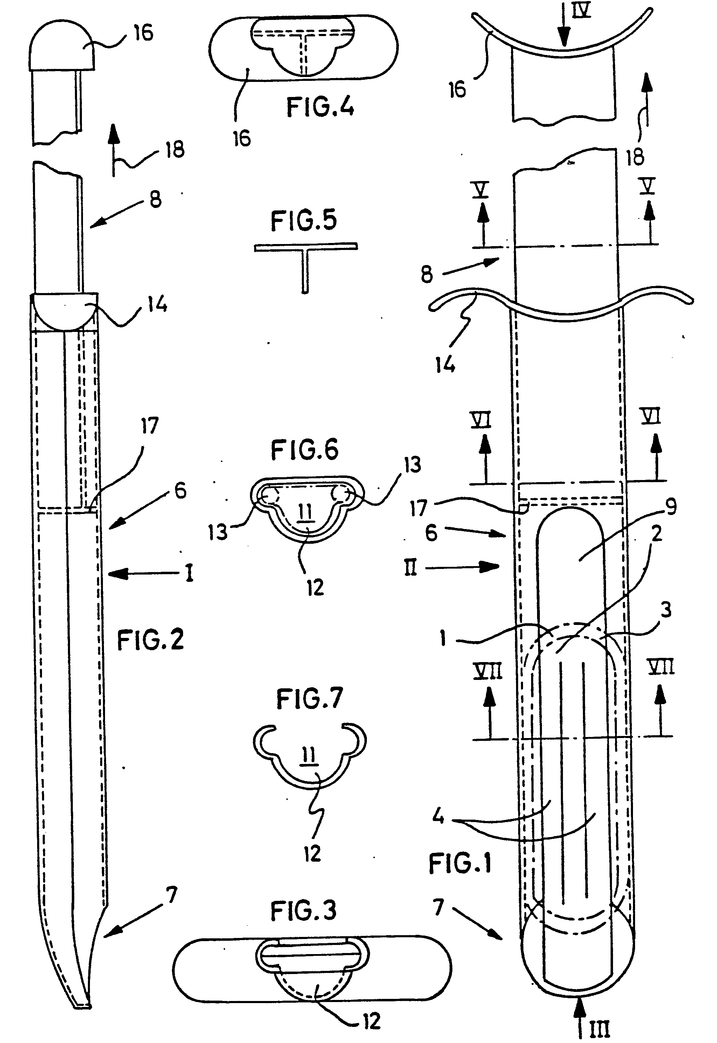

Die Fig. 1 bis 7 zeigen an einem Ausführungsbeispiel eine erste Variante einer Vorrichtung zum Einführen und Plazieren eines in Fig. 1 mit strichpunktierten Linien angedeuteten Diaphragmas 1, welches im Bevorratungs- und Gebrauchszustand in der Draufsicht kreisförmig ist und aus einer elastischen Membran 2 besteht, die an ihrem Rand mittels eines umlaufenden Wulstes 3 verstärkt ist. In einem seitlich zusammengedrückten Zustand, wie er in Fig. 1 dargestellt ist und weiter unten noch weiter erläutert wird, bilden sich selbsttätig zwei Taschen 4.

Die Vorrichtung besteht aus einem hohlen Einführstab 6, der an seinen beiden Enden offen ist, und einem dem Einführende 7 des Einführstabes 6 abgekehrten Ende angeordneten Kolben 8, der relativ zum Einführstab 6 verschiebbar ist, wie dieses weiter unten noch erläutert ist.

Der Mantel des Einführstabes 6 ist an seinem das Einführende 7 aufweisenden Endabschnitt mit einem sich in Längsrichtung des Einführstabes 6 erstreckenden Durchgangsschlitz 9 versehen, der zum Einführende 7 hin erweitert ausgebildet ist, wobei das Einführende 7 auf der dem Durchgangsschlitz 9 gegenüberliegenden Seite gekrümmt ausgebildet ist, wie insbesondere aus Fig. 2 erkennbar ist. Der lichte Querschnitt 11 des Einführstabes 6 ist hutförmig ausgebildet, wie insbesondere aus den Fig. 3, 4, 6 und 7 erkennbar ist. Dabei ist sowohl der Bodenbereich 12 des Querschnittes 11 abgerundet, wie auch der (dem "Hutrand" entsprechende) Randbereich 13.

An seinem dem Einführende 7 abgekehrten Ende ist der Einführstab 6 mit einer Handhabe 14 versehen, die so ausgebildet ist, daß beim Herausziehen des Einführstabes gegen den Kolben 8 der Zeige- und der Mittelfinger an der Handhabe 14 angreifen können, während sich der Handballen an einer Handhabe 16 des kolbenartigen Einsatzes 8 abstützen kann.

Der Kolben 8 ist bei dem in Fig. 1 bis 7 dargestellten Ausführungsbeispiel innerhalb seiner Handhabe 16 zunächst T-förmig (s. Fig. 5) und endet mit einer Stirnfläche 17, deren Kontur dem lichten Querschnitt 11 des Einführungsstabes 6 entspricht.

Die Arbeits- und Wirkungsweise der erfindungsgemäßen Vorrichtung gemäß den Fig. 1 bis 7 ist wie folgt:

Zunächst wird ein Diaphragma 1 in der in Fig. 1 mit strichpunktierten Linien dargestellten, seitlich zusammengerückten Weise vom Einführende 7 her in den Hohlraum des Einführstabes 6 eingeführt, wobei mithin der wulstförmige Rand 3 des Diaphragmas 1 im Randbereich 13 des Querschnittes 11 des Einzelstabes 6 geführt ist und die Taschen 4 in den Bodenbereich 12 des Querschnittes 11 erstreckt sind, wie dieses in Fig. 6 mit strichpunktierten Linien angedeutet ist. Erst dann wird das Diaphragma 1 mittels einer Tube oder dgl. mit einem spermiziden Mittel bestrichen, ohne daß die Benutzerin hierbei mithin mit dem Mittel selbst in Berührung kommt. Bei dieser Präparierung befindet sich der Kolben 8 in einer so weit zurückgezogenen Stellung, daß das Diaphragma 1 in der beschriebenen Weise in den Einführstab 6 eingeführt werden kann (s. Fig. 1 und 2).

Sodann kann die Vorrichtung mit dem in ihr enthaltenen Diaphragma eingeführt werden, wobei der Durchgangsschlitz 9 oben liegt. Das Einführen wird durch die Krümmung des Einführstabes 6 am Einführende 7 (s. Fig. 2) erleichtert.

Nach einem vollständigen Einführen des Einführstabes 6 wird sodann der Einführstab 6 gegen den Kolben 8 in Richtung des Pfeiles 18 zurückgezogen. Dabei nähert sich mithin die dem Einführende 7 zugekehrte Stirnfläche 17 des Kolbens 8 dem Diaphragma 1 und schiebt dieses aus dem Einführstab 6 hinaus. Da das Diaphragma 1 hierbei richtig plaziert ist, erfolgt eine zwangsläufig richtige Plazierung vor der Gebärmutter, ohne daß es hierfür weiterer Manipulationen bedarf. Denn nach dem Verlassen des Einführstabes 6 entfaltet sich das Diaphragma 1 aufgrund der Elastizität des Wulstes 3 von selbst in seine Ausgangsform.

Es ist ohne weiteres einsichtig, daß das spermizide Mittel lediglich dort zur Entfaltung kommt, wo es tatsächlich erwünscht ist, und daß bei zwangsläufig ordnungsgemäßer Handhabung der Vorrichtung keinerlei Verschmutzung der Umgebung erfolgt.

Die Vorrichtung ist nach entsprechender Reinigung beliebig häufig wiederverwendbar.

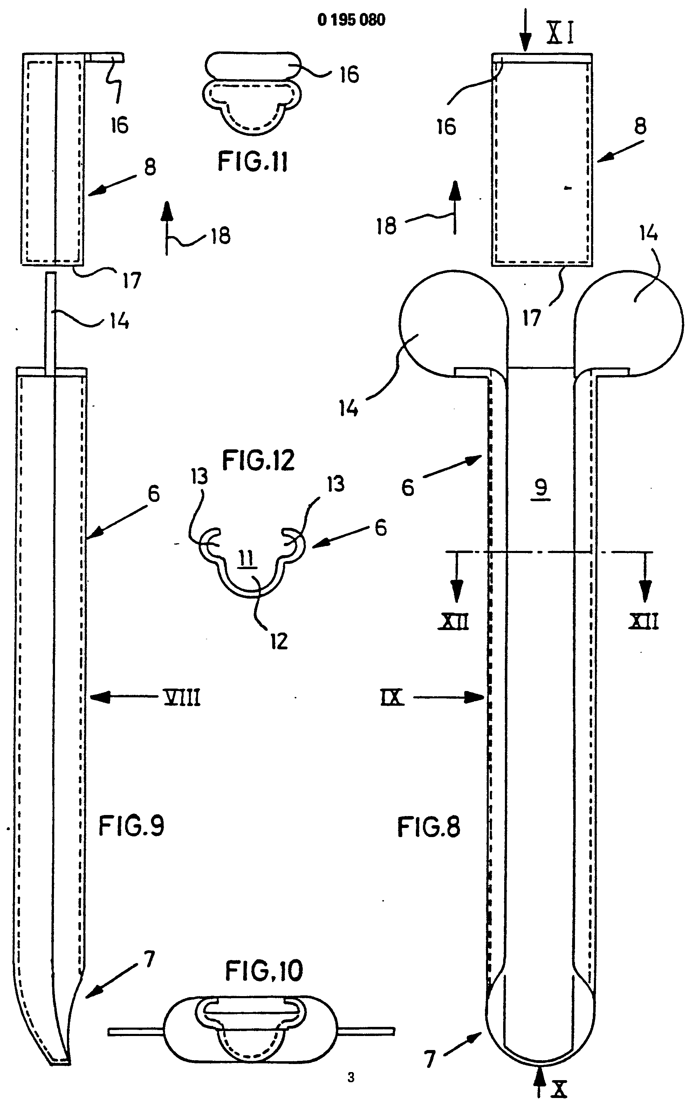

Die Fig. 8 bis 12 zeigen eine Variante, bei welcher gleiche bzw. gleichwirkende Teile mit gleichen Bezugszeichen versehen sind wie bei der Ausgestaltung gemäß den Fig. 1 bis 7, wobei sich bei dieser Variante der Durchgangsschlitz 9 über die gesamte Länge des Einführstabes 6 erstreckt. Dieses hat u. a. zur Folge, daß der Kolben 8 mehr oder weniger beliebig kurz ausgeführt werden kann, da er beim Herausziehen des Einführstabes 6 lediglich mit einem Finger relativ zu dem Einführstab 6 zu halten ist und sich dieser Finger ohne weiteres in den Durchgangsschlitz 9 hineinerstrecken kann.

Die Handhabe 14 des Einführstabes 6 ist bei dieser Variante in der Art zweier Ohren ausgebildet, was die ordnungsgemäße Handhabung noch weiterhin unterstützt, nämlich nach dem Einführen des Einführstabes 6 diesen relativ zum feststehenden Einsatz 8 herauszuziehen und nicht umgekehrt den Kolben 8 relativ zu dem noch festgehaltenen eingeführten Einführstab 6 zum Einführende 7 hin nach vorn zu bewegen.

Neben den bereits genannten und weiteren Vorteilen sei noch angemerkt, daß sich die Ausgestaltung gemäß den Fig. 8 bis 12 besonders leicht reinigen läßt, und zwar beispielsweise mittels eines im lichten Querschnitt 11 des Einführstabes 6 angepaßten Reinigungskörper wie einer Bürste oder dgl.

## Patentansprüche

1. Vorrichtung zum Einführen eines Diaphragmas (1) in eine Scheide sowie zum Plazieren des Diaphragmas vor der Gebärmutter, bestehend aus einem hohlen Einführstab (6), an dessen in Längsrichtung geschlitzt (9) ausgebildetem offenen Einführende (7) das Diaphragma lösbar zu befestigen ist, und in dem ein relativ zum Einführstab verschiebbarer Kolben (8) angeordnet ist, dadurch gekennzeichnet, daß

a) der lichte Querschnitt (11) des Einführstabes (6) durch einen abgerundeten Boden (12) und einen hieran anschließenden, verbreiterten abgerundeten Randbereich (13) zum geführten Halten des Diaphragma-Rundwulstes (3) hutförmig ausgebildet ist, und

b) der Einführstab (6) mit einem einzigen, im verbreiterten Randbereich (13) angeordneten Schlitz (9) versehen ist.

2. Vorrichtung nach Anspruch 1, dadurch gekennzeichnet, daß sich der Schlitz (9) über die gesamte Länge des Einführstabes (6) erstreckt.

3. Vorrichtung nach Anspruch 1 oder 2, dadurch gekennzeichnet, daß der Schlitz (9) zum Einführende (7) des Einführstabes (6) hin erweitert ausgebildet ist.

4. Vorrichtung nach einem oder mehreren der vorhergehenden Ansprüche, dadurch gekennkeichnet, daß das Einführende (7) des Einführstabes (6) gekrümmt ausgebildet ist.

5. Vorrichtung nach einem oder mehreren der vorhergehenden Ansprüche, dadurch gekennzeichnet, daß die Querschnittsform des Kolbens (8) der lichten Querschnittsform des Einführstabes (6) entspricht.

## Claims

1. Device for introducing a diaphragm (1) into the vagina and for placement of the diaphragm in front of the womb, comprising a hollow insertion rod (6), the diaphragm being detachably fixable to the open, lengthwise slotted (9) insertion end (7) of said rod (6), and a piston (8) being disposed in said rod (6) to be movable in relation thereto, characterised in that

a) a rounded base (12) and a broadened, rounded rim section (13) continuous therewith give the inside cross-section (11) of the insertion rod (6) the shape of a hat to guide the circular rim (3) of the diaphragm while gripped, and

b) the insertion rod (6) is provided with a single slot (9), disposed in the broadened rim section (13).

2. Device according to Claim 1, characterised in that the slot (9) extends the whole length of the insertion rod (6).

3. Device according to Claim 1 or 2, characterised in that the slot (9) is enlarged towards the insertion end (7) of the insertion rod (6).

4. Device according to one or more of the preceding claims, characterised in that the insertion end (7) of the insertion rod (6) is curved.

5. Device according to one or more of the preceding claims, characterised in that the cross-section of the piston (8) corresponds in shape to the inside cross-section of the insertion rod (6).

## Revendications

1. Dispositif pour l'introduction d'un diaphragme (1) dans un vagin et pour le placement de ce diaphragme devant l'utérus, du genre comportant un barreau d'insertion (6) creux à l'extrémité (7) à introduire ouverte présentant une fente longitudinale (9) à laquelle le diaphragme est à fixer de façon amovible, et dans lequel un piston (8) déplaçable par rapport au barreau d'insertion est disposé, caractérisé en ce que:

A) la section ouverte (11) du barreau d'insertion (6) présente une forme de chapeau ayant un fond arrondi (12) et une zone périphérique (13) bombée raccordée latéralement audit fond en l'élargissant en vue de maintenir en le guidant le bourrelet (3) du diaphragme,

B) le barreau d'insertion (6) est pourvu dans sa zone périphérique (13) qui l'élargit, d'une seule et unique fente (9).

2. Dispositif conforme à la revendication 1, caractérisé en ce que la fente (9) s'étend sur toute la largeur du barreau d'insertion (6).

3. Dispositif conforme à l'une quelconque des revendications 1 ou 2, caractérisé en ce que la fente (9) s'étend jusqu'à l'extrémité (7) à introduire du barreau d'insertion (6).

4. Dispositif conforme à l'une quelconque des revendications précédentes, caractérisé en ce que l'extrémité (7) à introduire du barreau d'insertion (6) a une forme courbe.

5. Dispositif conforme à l'une quelconque ou à plusieurs des revendications précédentes, caractérisé en ce que la forme de la section droite du piston (8) correspond à la forme de la section droite ouverte du barreau d'insertion (6).

0 195 080

FIG.4

FIG.5

FIG.6

FIG.7

FIG.3

FIG.2

FIG.1

FIG.11

FIG.12

FIG.9

FIG.10

FIG.8

3